Europäisches Patentamt

(19) European Patent Office    (11) Publication number: **0 189 282**

Office européen des brevets                                **B1**

(12)                EUROPEAN PATENT SPECIFICATION

(45) Date of publication of the patent specification:        (51) Int. Cl.⁴: **C 07 C 11/02,** C 07 C 5/48,
29.03.89                                                      B 01 J 27/18

(21) Application number: **86300292.9**

(22) Date of filling: **17.01.86**

(54) The production of mono-olefins by the oxidative dehydrogenation of lower paraffins.

(30) Priority: **19.01.85 GB 8501401**

(43) Date of publication of application:
**30.07.86 Bulletin 86/31**

(45) Publication of the grant of the patent:
. **29.03.89 Bulletin 89/13**

(84) Designated Contracting States:
**BE DE FR GB IT NL**

(56) References cited:
**US-A-3 119 883**
**US-A-3 775 508**
**US-A-3 927 138**
**US-A-4 252 680**

(73) Proprietor: **NATIONAL RESEARCH DEVELOPMENT
CORPORATION, 101 Newington Causeway,
London SE1 6BU (GB)**

(72) Inventor: **Harrison, Philip Geoffrey, 24 Delville
Avenue, Keyworth Nottinghamshire, NG12 5JA
(GB)**
Inventor: **Argent, Alec, 15 North Market Road,
Great Yarmouth Norfolk, NR30 2DX (GB)**

(74) Representative: **Hamilton, Raymond, British
Technology Group 101 Newington Causeway,
London SE1 6BU (GB)**

## Description

The present invention relates in general to the production of a mono-olefin by the oxidative dehydrogenation of the corresponding paraffin. In particular, the invention relates to a process in which a tin oxide/phosphorus oxide catalyst is used in the oxidative dehydrogenation of ethane to ethylene, propane to propylene or isobutane to isobutene.

Historically, the oxidative dehydrogenation of hydrocarbons has been approached along two different paths which occasionally overlap. The first approach is the oxidative dehydrogenation of a mono-olefin to produce a di-olefin. Thus, US Patent No. 3 274 283 published in 1961 describes the dehydrogenation of butene-1, buten-2, 2-methyl-buten-1 and 2-methyl-buten-2 using as catalyst phosphoric acid deposited on tin oxide. The intended product of this process was a di-olefin. There followed a number of patent publications extending and improving the aforesaid tin/phosphorus catalysts for the dehydrogenation of alkenes, cycloalkenes, alkyl pyridines and alkyl aromatic hydrocarbons. An early example of such a patent is US Patent No. 3 320 329 published in 1965. The object of this approach was to produce di-olefins, such as butadiene and isoprene, as monomers for subsequent polymerisation purposes.

The second approach involved the oxidative dehydrogenation of paraffins, also to produce di-olefins for the same purpose. The dehydrogenation of paraffins was first achieved using Group VIII metal catalysts at relatively high loadings (20 - 75% wt), although vanadium and molybdenum were subsequently used. The earliest patent adopting this approach known to the Applicant is US Patent No. 3 502 739 published in 1967. This patent clearly distinguishes between the paraffin dehydrogenation step requiring a Group VIII metal and the olefin dehydrogenation step, for which the preferred catalyst is that described in the aforesaid US Patent No. 3 320 329.

A logical sequel to the two approaches was embodied in US Patent No. 3 781 223 published in 1971, in which they are combined into a single step. However, the intention to make reactive di-olefins as polymer precursors remained. This objective is reflected in the initial choice of $C_4$ to $C_{12}$ paraffins as feedstock. Later patents, however, extend the feed hydrocarbon to $C_3$ - $C_{20}$ paraffins (USP-3 801 671) and $C_2$ - $C_{12}$ paraffins (USP-3 927 138), for the purpose of producing di-olefins. The catalysts used for this purpose are composite catalysts incorporating at least two metallic components. A desirable objective would be to convert ethane, propane or isobutane in a single step to mono-olefins.

It has now been found that a catalyst consisting essentially of tin oxide and phosphorus oxide ie tin oxide and phosphorus oxide substantially free from metallic promoters capable of promoting the conversion of mono-olefins to di-olefins, can surprisingly be used to selectively catalyse the oxidative dehydrogenation of ethane, propane or isobutane to ethylene, propylene and isobutene respectively.

Accordingly, the present invention provides a process for the production of a mono-olefin by the oxidative dehydrogenation of the corresponding paraffin which process comprises reacting either ethane, propane or isobutane and an oxygen-containing gas at elevated temperature in the presence of a catalyst consisting essentially of tin oxide and phosphorus oxide.

The ethane, propane or isobutane may be obtained from any onvenient source. They may suitably be used in the substantially pure form or in the form of mixtures, which may contain for example paraffins other than ethane, propane or isobutane. Oxidative dehydrogenation of ethane, propane and isobutane produces respectively ethylene, propylene and isobutene.

It is believed that the activity and/or selectivity of tin oxide/phosphorus oxide catalysts depends to some extent on their method of preparation. A method of preparation which has been found to produce active and selective catalysts comprises either sequential hydrolysis or co-hydrolysis of hydrolysable tin and phosphorus compounds. Preferred sources of hydrolysable tin and phosphorus compounds are tin (IV) compounds, i.e. stannic compounds, and phosphorus (V) compounds, since these compounds produce on hydrolysis stannic oxide and phosphorus pentoxide respectively. Preferred tin (IV) compounds are the tin tetrahalides and tin tetraalkyls and preferred phosphorus (V) compounds are the phosphorus pentahalides and the phosphorus oxyhalides.

Preferably the molar ratio of Sn:P in the finished catalyst is less than 2 in order to ensure the most selective catalysts. Catalysts which have higher levels of tin tend to produce higher yields of unwanted by-products, such as methane.

The oxygen-containing gas may suitably be air, though gases richer or poorer than air with regard to their oxygen content, for example oxygen itself, may be employed.

The process is preferably carried out under continuous flow conditions. The elevated temperature may suitably be in the range from 200 to 700°C. The pressure may suitably be in the range from 1 to 50 bar.

Typically, the catalyst may be charged to a reactor under ambient conditions and the reactor slowly heated to the desired reaction temperature under a continuous flow of the oxygen-containing gas. At the reaction temperature, the flow of oxygen-containing gas may be replaced by a gas flow containing both the paraffin and the oxygen-containing gas.

The products of the reaction may subsequently be fed to a recovery unit wherein the gaseous reactants and product may be separated using known techniques.

The invention will now be further illustrated by reference to the following Examples.

2

**Catalyst preparation**

The tin oxide/phosphorus oxide catalysts were prepared by dissolving the appropriate amount of tin tetrachloride in water sufficient to produce a solution 4 molar in tin. After this stage was complete sufficient phosphoryl chloride was added to the solution to produce a mixture of the desired Sn:P ratio. The resulting solution was brought to pH 4 by the addition of aqueous .880 ammonia during which time the solution gelled. The gel was centrifuged, redispersed in water until all chloride was removed, treated with IN nitric acid and dried in air at 120°C.

**Catalyst run details - examples 1 to 12**

3 ml of the catalyst was packed into a stainless steel tube reactor (6 mm internal diameter). The reactor was then slowly heated to 550°C under a continuous flow of air. As soon as the catalyst reached 550°C, a gaseous feed comprising 50 % by volume ethane and 50 % by volume air was passed over the catalyst. The gaseous products were analysed by gas chromatography.

The experimental results obtained for two catalysts are given in the Table. Examples 1 - 4 relate to a catalyst having a Sn:P molar ratio of 1 : 0.625 and a feed gas flow rate of 6 mls$^{-1}$, Examples 5 - 8 relate to the same catalyst at a feed gas flow rate of 33 mls$^{-1}$ and Examples 9 - 12 relate to a catalyst having a Sn:P molar ratio of 1: 0.113 and a feed gas flow rate of 33 mls$^{-1}$.

**TABLE**

| Example | Feed Gas Pressure (bar) | % Ethane Conversion (molar) | % Selectivity (molar) | | | |
|---|---|---|---|---|---|---|
| | | | CO | $CO_2$ | $CH_4$ | $C_2H_4$ |
| 1 | 6.6 | 30.4 | - | - | 15.1 | 84.6 |
| 2 | 13.2 | 64.3 | - | 2.0 | 4.6 | 93.3 |
| 3 | 19.8 | 48.8 | - | - | 6.1 | 93.9 |
| 4 | 26.4 | 27.1 | 5.2 | 8.5 | 10.5 | 75.7 |
| 5 | 6.6 | 21.3 | - | 7.2 | 12.4 | 79.7 |
| 6 | 13.2 | 53.9 | - | 2.4 | 4.0 | 92.0 |
| 7 | 19.8 | 37.4 | - | - | 5.6 | 91.3 |
| 8 | 26.4 | 48.5 | - | 1.2 | 3.5 | 92.1 |
| 9 | 6.6 | 40.0 | 4.7 | 0.5 | 14.7 | 75.9 |
| 10 | 13.2 | 17.6 | - | 1.7 | 18.0 | 78.7 |
| 11 | 19.8 | 21.8 | 3.1 | 4.8 | 16.1 | 73.3 |
| 12 | 26.4 | 14.5 | 6.2 | 8.1 | 19.4 | 62.0 |

**Claims**

1. A process for the production of a mono-olefin by the oxidative dehydrogenation of the corresponding paraffin which process comprises reacting either ethane, propane or isobutane and an oxygen-containing gas at elevated temperature in the presence of a catalyst consisting of tin oxide and phosphorus oxide.

2. A process according to claim 1 wherein the catalyst is obtained by a process comprising either sequential hydrolysis or co-hydrolysis of hydrolysable tin and phosphorus compounds.

3. A process according to claim 2 wherein the hydrolysable tin compound is a tin (IV) compound.

4. A process according to claim 3 wherein the tin (IV) compound is either a tin tetrahalide or a tin tetraalkyl.

5. A process according to claim 2 wherein the hydrolysable phosphorus compound is a phosphorus (V) compound.

6. A process according to claim 5 wherein the phosphorus (V) compound is either a phosphorus pentahalide or a phosphorus oxyhalide.

7. A process according to claim 1 wherein the molar ratio of Sn:P is less than 2.

8. A process according to claim 1 wherein the oxygen-containing gas is air.

9. A process according to claim 1 wherein the elevated temperature is in the range from 200 to 700°C.

10. A process according to claim 1 wherein ethane is reacted with an oxygen-containing gas at elevated temperature in the presence of a catalyst consisting essentially of tin oxide and phosphorus oxide to produce ethylene.

# EP 0 189 282 B1

## Patentansprüche

1. Verfahren zur Herstellung eines Monoolefins durch oxidative Dehydrierung des entsprechenden Paraffins, wobei dieses Verfahren darin besteht, entweder Ethan, Propan oder Isobutan und ein Sauerstoff-enthaltendes Gas bei erhöhter Temperatur in Gegenwart eines Katalysators umzusetzen, der aus Zinnoxid und Phosphoroxid besteht.

2. Verfahren nach Anspruch 1, wobei der Katalysator erhalten wird nach einem Verfahren, welches eine aufeinander folgende Hydrolyse oder eine Cohydrolyse von hydrolysierbaren Zinn- und Phosphorverbindungen vorsieht.

3. Verfahren nach Anspruch 2, wobei die hydrolysierbare Zinnverbindung eine Zinn(IV)-Verbindung ist.

4. Verfahren nach Anspruch 3, wobei die Zinn(IV)-Verbindung entweder ein Zinntetrahalogenid oder ein Zinntetraalkyl ist.

5. Verfahren nach Anspruch 2, wobei die hydrolysierbare Phosphorverbindung eine Phosphor(V)-Verbindung ist.

6. Verfahren nach Anspruch 5, wobei die Phosphor(V)-Verbindung entweder ein Phosphorpentahalogenid oder ein Phosphoroxyhalogenid ist.

7. Verfahren nach Anspruch 1, wobei das Molverhältnis Sn:P weniger als 2 beträgt.

8. Verfahren nach Anspruch 1, wobei das sauerstoffenthaltende Gas Luft ist.

9. Verfahren nach Anspruch 1, wobei die erhöhte Temperatur zwischen 200 und 700°C liegt.

10. Verfahren nach Anspruch 1, wobei Ethan mit einem Sauerstoff enthaltenden Gas bei erhöhter Temperatur in Gegenwart eines Katalysators, der im wesentlichen aus Zinnoxid und phosphoroxid besteht, zur Erzeugung von Ethylen umgesetzt wird.

## Revendications

1. Procédé de production d'une mono-oléfine par la déshydrogénation oxydante de la paraffine correspondante, ce procédé comprenant la réaction de l'éthane, du propane ou de l'isobutane, d'une part, et d'un gaz contenant de l'oxygène, d'autre part, à température élevée en présence d'un catalyseur consistant en de l'oxyde d'étain et en de l'oxyde de phosphore.

2. Procédé selon la revendication 1, dans lequel le catalyseur est obtenu par un procédé comprenant l'hydrolyse, en opérations sucessives, ou la cohydrolyse, de composés hydrolysables de l'étain et du phosphore.

3. Procédé selon la revendication 2, dans lequel le composé hydrolysable de l'étain est un composé d'étain (IV).

4. Procédé selon la revendication 3, dans lequel le composé d'étain (IV) est un tétrahalogénure d'étain ou un étain-tétraalkyle.

5. Procédé selon la revendication 2, dans lequel le composé hydrolysable du phosphore est un composé de phosphore-(V).

6. Procédé selon la revendication 5, dans lequel 1e composé de phosphore (V) est un pentahalogénure de phosphore ou un oxyhalogéure de phosphore.

7. Procédé selon la revendication 1, dans lequel le rapport molaire de Sn:P est inférieur à 2.

8. Procédé selon la revendication 1, dans lequel le gaz contenant de l'oxygène est de l'air.

9. Procédé selon la revendication 1, dans lequel la température élevée se situe dans l'intervalle allant de 200 à 700°C.

10. Procédé selon la revendication 1, dans lequel on fait réagir l'éthane avec un gaz contenant de l'oxygène, à température élevée, en présence d'un catalyseur consistant essentiellement en de l'oxyde d'étain et de l'oxyde de phosphore, pour produire de l'éthylène.